(19) Europäisches Patentamt
European Patent Office
Office européen des brevets

(11) Veröffentlichungsnummer: **0 133 578**
**B1**

(12) # EUROPÄISCHE PATENTSCHRIFT

(45) Veröffentlichungstag der Patentschrift: **01.08.90**

(51) Int. Cl.⁵: **C 07 D 207/20**, A 61 K 31/40

(21) Anmeldenummer: **84109462.6**

(22) Anmeldetag: **08.08.84**

(54) **3-Aryl-3-pyrrolin-Derivate, Verfahren zu deren Herstellung sowie diese enthaltende Arzneimittel für die Bekämpfung von Herz- und Gefässkrankheiten.**

(30) Priorität: **09.08.83 DE 3328643**

(43) Veröffentlichungstag der Anmeldung:
**27.02.85 Patentblatt 85/09**

(45) Bekanntmachung des Hinweises auf die Patenterteilung:
**01.08.90 Patentblatt 90/31**

(84) Benannte Vertragsstaaten:
**AT BE CH DE FR GB IT LI LU NL SE**

(56) Entgegenhaltungen:
**CH-A- 556 336**
**THE JOURNAL OF ORGANIC CHEMISTRY, Band 39, Nr. 25, 1974, Seiten 3781-3783, *3782, Formal 3; Spalte 2, Zeilen 44-59, Columbus, Ohio, US; A.G. HORTMANN et al.: "A new route to 2-vinylaziridines and an unusual intramolecular analog of the SN2' reaction leading to aziridine ring formation"**

**Die Akte enthält technische Angaben, die nach dem Eingang der Anmeldung eingereicht wurden und die nicht in dieser Patentschrift enthalten sind.**

(73) Patentinhaber: **GÖDECKE AKTIENGESELLSCHAFT**
**Salzufer 16**
**D-1000 Berlin 10 (DE)**

(72) Erfinder: **Satzinger, Gerhard, Dr.**
**Im Mattenbühl 7**
**D-7809 Denzlingen (DE)**
Erfinder: **Mannhardt, Karl, Dr.**
**Pfauenstrasse 14**
**D-7807 Elzach-Oberprechtal (DE)**
Erfinder: **Hartenstein, Johannes, Dr.**
**Fohrenbühl 23**
**D-7801 Stegen-Wittental (DE)**
Erfinder: **Herrmann, Manfred, Dr.**
**Wolfweg 25**
**D-7811 St. Peter (DE)**
Erfinder: **Fritschi, Edgar, Dr.**
**Am Scheuerwald 2**
**D-7811 St. Peter (DE)**
Erfinder: **Steinbrecher, Wolfgang, Dr.**
**Schwarzwaldstrasse 16**
**D-7803 Gundelfingen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 133 578 B1

Courier Press, Leamington Spa, England.

**Beschreibung**

Gegenstand der vorliegenden Erfindung sind 3-Aryl-3-pyrolin-Derivate der allgemeinen Formel I

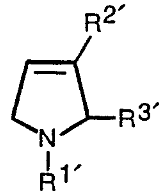

(I)

in welcher $R^{1'}$ ein Wasserstoffatom, eine geradkettige oder verzweigte Alkylgruppe mit bis zu 4 Kohlenstoffatomen oder einen Benzylrest, $R^{2'}$ einen unsubstituierten oder durch bis zu 3 gleiche oder verschiedene Substituenten, nämlich Alkyl- oder Alkoxyreste mit bis zu 4 Kohlenstoffatomen oder mit Halogenatomen, Nitro-, Trifluormethyl- oder Hydroxylgruppen substituierten Phenylrest und $R^{3'}$ ein Wasserstoffatom oder eine geradkettige oder verzweigte Alkylgruppe bis zu 4 Kohlenstoffatomen bedeutet, sowie deren pharmakologisch verträgliche Salze mit organischen oder anorganischen Säuren; mit Ausnahme derjenigen Verbindungen, bei denen $R^{1'}$ und $R^{3'}$ ein Wasserstoffatom und gleichzeitig $R^{2'}$ einen unsubstituierten Phenylrest oder einen 3-Trifluormethylphenylrest darstellt.

Aus der Gruppe der Verbindungen gemäß der allgemeinen Formel I sind bereits zwei Vertreter bekannt, nämlich das 3-Phenyl-3-pyrrolin [J. Org. Chem. *39*, S. 3781 (1974)] und das 3-(3-Trifluormethylphenyl)-3-pyrrolin [DE—OS 2017255]. Diese Verbindungen sind jedoch nur als Zwischenprodukte beschrieben.

Die Verbindungen der allgemeinen Formel I sind überraschend pharmakologisch wirksam und zeichnen sich durch wertvolle kardiovaskuläre Eigenschaften aus, so daß sie insbesondere bei der Therapie der Herzmuskelschwäche und des hypotonen Formenkreises eingesetzt werden können.

Ein weiterer Gegenstand der vorliegenden Erfindung sind daher Arzneimittel mit kardiovaskulärer Wirkung, welche dadurch gekennzeichnet sind, daß sie neben üblichen Hilfs- und Trägerstoffen mindestens eine Verbindung der allgemeinen Formel II

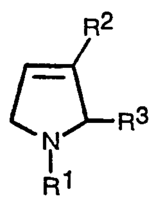

(II),

in welcher $R^1$ ein Wasserstoffatom, eine geradkettige oder verzweigte Alkylgruppe mit bis zu 4 Kohlenstoffatomen oder einen Benzylrest, $R^2$ einen unsubstituierten oder durch bis zu 3 gleiche oder verschiedene Substituenten, nämlich Alkyl- oder Alkoxyreste mit bis zu 4 Kohlenstoffatomen oder mit Halogenatomen, Nitro-, Trifluormethyl- oder Hydroxylgruppen substituierten Phenylrest und $R^3$ ein Wasserstoffatom oder eine geradkettige oder verzweigte Alkylgruppe mit bis zu 4 Kohlenstoffatomen bedeutet,

oder pharmakologisch verträgliche Salze derselben mit organischen oder anorganischen Säuren enthalten.

Der Phenylrest der Formel I und II kann bis zu 3 gleiche oder verschiedene Substituenten, nämlich insbesondere Alkyl- und Alkoxyreste mit bis zu 4 Kohlenstoffatomen, bevorzugt Methyl- oder Methoxygruppen sowie Halogenatome, wie Chlor, Brom oder Fluor oder eine Nitro-, Trifluormethyl oder Hydroxymethyl- oder eine Hydroxylgruppe enthalten. Zwei benachbarte Reste können auch eine Methylendioxy-Gruppe bilden. $R^3$ kann z.B. Wasserstoff, eine n-Butyl, Isobutyl, n-Propyl-, Isopropyl und bevorzugt eine Methyl- oder Ethylgruppe sein.

Die Verbindungen der allgemeinen Formeln I und II können dadurch hergestellt werden, daß man in an sich bekannter Weise entweder

a) eine Verbindung der allgemeinen Formel III

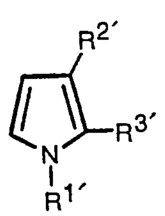

(III),

in welcher $R^1$, $R^2$ und $R^3$ die obengenannte Bedeutung haben, mittels Zink und einer Mineralsäure reduziert oder

b) eine Verbindung der allgemeinen Formel IV

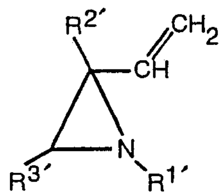

(IV),

in welcher $R^1$, $R^2$ und $R^3$ die obengenannte Bedeutung haben, mit Bromwasserstoffsäure umsetzt oder

c) eine Verbindung der allgemeinen Formel V

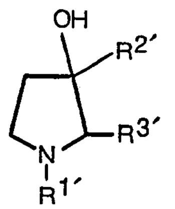

(V),

in welcher $R^1$, $R^2$ und $R^3$ die obengenannte Bedeutung haben, mit einer Mineralsäure dehydratisiert, und die erhaltenen Verbindungen der Formel I bzw. II gewünschtenfalls in an sich bekannter Weise anschließend in deren pharmakologisch verträgliche Salze überführt.

Das Verfahren a) ist in J. Am. Chem. Soc. *51*, S. 889—890 (1929) beschrieben. Als anorganische Säure wird bevorzugt Salzsäure eingesetzt. Die Reaktion sollte unter guter Kühlung ablaufen. Am besten tropft man zu einer Lösung der Verbindung III in einem inerten Lösungsmittel, wie z.B. Tetrahydrofuran oder Methanol, in dem vorgelegter Zinkstaub dispergiert ist, unter gutem Rühren und Eiskühlung bei 10—15°C, konzentrierte Salzsäure und läßt das Gemisch unter Kühlung bei 0—5°C noch einige Zeit nachreagieren.

Die Verbindungen II lassen sich in üblicher Weise alkalisch extrahieren.

Die Ausgangsprodukte der allgemeinen Formel III lassen sich nach der Vorschrift Journ. prakt. Chem. *314*, S. 355 (1972) sowie Chemical Abstracts *49*, 10 838 g (1955) herstellen.

Das Verfahren b) ist in J. Org. Chem. *39*, S. 3781 (1974) beschrieben. Die Verbindungen der allgemeinen Formel IV werden im System HBr—$H_2O$—THF bei leicht erhöhter Raumtemperatur, vorzugsweise bei 25—30°C umgelagert. Hierbei werden die Verbindungen der allgemeinen Formel I bzw. II in einer Ausbeute von etwa 70% gewonnen. Am besten setzt man etwa 48%ige Bromwasserstoffsäure mit einer Dichte von etwa 1,5 ein. Die Reaktion wird durchgeführt, indem man einen geringen molaren Überschuß von Bromwasserstoffsäure in etwa der zwanzigfachen Gewichtsmenge Tetrahydrofuran löst und dieses Gemisch langsam zu einer Lösung der Verbindungen IV in Tetrahydrofuran gibt. Nach etwa 6 Stunden ist die Reaktion in der Regel beendet und die Hydrobromide der Verbindungen der Formel II können in üblicher Weise durch Einengen des Reaktionsgemisches und Umkristallisation aus polaren Lösungsmitteln, wie z.B. Acetonitril rein erhalten werden. Die Freisetzung der Verbindungen I bzw. II erfolgt dann mit Natriumcarbonat und Extraktion mittels Methylenchlorid.

Die Ausgangsprodukte der allgemeinen Formel IV werden nach der Vorschrift J. Org. Chem. *39*, S. 3781 (1974) aus entsprechenden Azabicyclobutanen erhalten. Diese wiederum sind allgemein zugänglich nach der Vorschrift J. Amer. Chem. Soc. *94*, S. 2758 (1972) und J. Org. Chem. *33*, S. 2121 (1968).

Das Verfahren c) ist in der DE—OS 2017255 beschrieben. Die Verbindungen der allgemeinen Formel V sind durch Grignard-Reaktion zugänglich und ebenfalls beschrieben. [J. Med. Pharm. Chem. *7*, S. 60 (1964)]. Die Dehydratisierung erfolgt, indem man die Verbindungen V in konzentrierter Mineralsäure, vorzugsweise Salzsäure, 10 bis 20 Stunden erhitzt. Vorzugsweise erfolgt die Reaktion bei Rückflußtemperatur. Die erhaltenen Hydrochloride können in üblicher Weise durch Umsetzung mit schwachen Basen, wie z.B. Alkalicarbonaten oder Ammoniak und anschließende Extraktion hergestellt werden.

Als physiologisch verträgliche Säuren kommen z.B. in Betracht Chlor-, Brom- oder Jodwasserstoffsäure, Schwefelsäure, Phosphorsäure, Toluolsulfonsäure, Benzolsulfonsäure, Sulfaminsäure, Fettsäuren, wie z.B. Essig-, Propion-, Butter-, Öl-, Palmitin- oder Stearinsäure, weiter Oxal-, Malon-, Bernsteinsäure, sowie Äpfel-, Wein-, Zitronen-, Fumar-, Malein-, Milch-, Glykol-, Brenztrauben-, Benzoe-, Salicyl- oder Mandelsäure.

Die Dosierung beim Menschen dürfte pro Dosis abhängig vom Schweregrad der Erkrankung oral etwa bei 5—50 mg und bei parenteraler Verabreichung etwa bei 1 mg bis 10 mg liegen.

Die erfindungsgemäßen Verbindungen der allgemeinen Formel I können in flüssiger oder fester Form oral oder parenteral appliziert werden. Als Injektionslösung kommt vor allem Wasser zum Anwendung, welches die bei Injektionslösungen üblichen Zusätze, wie Stabilisierungsmittel, Lösungsvermittler oder Puffer enthält.

Derartige Zusätze sind z.B. Tartrat- und Citrat-Puffer, Äthanol, Komplexbildner (wie Äthylendiamintetraessigsäure und deren nichttoxische Salze) sowie hochmolekulare Polymere (wie flüssiges Polyäthylenoxid) zur Viskositätsregulierung. Feste Trägerstoffe sind z.B. Stärke, Lactose, Mannit, Methylcellulose, Talkum, hochdisperse Kieselsäure, höhermolekulare Fettsäuren (wie Stearinsäure), Gelatine, Agar-Agar, Calciumphosphat, Magnesiumstearat, tierische und pflanzliche Fette, feste hochmolekulare Polymere (wie Polyäthylenglykol); für orale Applikation geeignete Zubereitungen können gewünschtenfalls zusätzliche Geschmacks- und/oder Süßstoffe enthalten.

Die folgenden Beispiele dienen der näheren Erläuterung der Erfindung:

## Beispiel 1

1-Methyl-3-phenyl-3-pyrrolin. (1a) Oxalat

Zu einer Lösung von 18,7 g (0,1 Mol) 1-Methyl-3-phenyl-pyrrol in 500 ml Methanol gibt man 40 g Zinkstaub und tropft unter Eiskühlung bei 10 bis 15°C 70 ml konzentrierte Salzsäure zu. Nach 1 Stunde kühlt man das Reaktionsgemisch auf 0 bis 5°C, saugt vom Zink ab, wäscht mit 150 ml 2N HCl und versetzt das Filtrat mit 2 l Wasser. Man stellt mit Natriumhydroxid alkalisch, extrahiert mit Toluol, wäscht mit Wasser, trocknet über wasserfreiem Natriumsulfat und zieht das Lösungsmittel im Vakuum ab. Die so erhaltene gereinigte Base (Schmp. 43 bis 46°C) wird in Ethylacetat gelöst und mit einer äquivalenten Menge Oxalsäure als Salz gefällt. Der Niederschlag wird abgesaugt und aus Acetonitril umkristallisiert. Man erhält 1-Methyl-3-phenyl-3-pyrrolin in Form farbloser Kristalle vom Schmp. 133 bis 137°C (aus Acetonitril). In analoger Weise erhält man *1-Methyl-3-methoxyphenyl)-3-pyrrolin*; (1b); Schmp. 132 bis 134°C aus Acetonitril.

Die Herstellung der Ausgangsverbindungen erfolgt gemäß Journ. prakt. Chem. *314*, S. 355 (1972) und Chem. Abstr. *49*, 10838 g (1955) Oxalat.

## Beispiel 2

3-(4-Methylphenyl)-3-pyrrolin (2a)

Zu einer unter Stickstoff gerührten Lösung von 15,9 g (0,1 Mol) 2-(4-Methylphenyl)-2-vinylaziridin in 250 ml Tetrahydrofuran tropft man bei Raumtemperatur innerhalb von 30 Minuten 18,5 g (0,11 Mol) einer wäßrigen Bromwasserstofflösung der Dichte 1,5. Man rührt weitere 3½ Stunden bei 30°C, saugt den ausgefallenen Niederschlag ab, wäscht mit Tetrahydrofuran/Ether 1:1 und kristallisiert aus Propanol-2 um. Man erhält farblose Nadeln von *3-(4-Methylphenyl)-3-pyrrolin·HBr* vom Schmp. 184 bis 185°C. In analoger Weise erhält man *3-(3-Trifluormethylphenyl)-3-pyrrolin·HBr* (2b) vom Schmp. 233 bis 236°C (aus Ethanol) und *3-(3-Methoxyphenyl)-3-pyrrolin·HBr* (2c) vom Schmp. 158 bis 159°C (aus Acetonitril) und *3-Phenyl-3-pyrrolin·Hbr* (2d) vom Schmp. 158 bis 161°C (aus Acetonitril).

Zur Herstellung der als Ausgangsprudukt verwendeten Vinylaziridine geht man aus von Methojodiden gemäß J. Org. Chem. *33*, S. 2121 (1968), die über Azirine [J. Am. Chem. Soc. *94*, S. 2758 (1972)] in Azabicyclobutane übergeführt werden. Letztere werden analog J. Org. Chem. *39*, S. 3781 (1974) zu Vinylaziridinen umgesetzt.

## Beispiel 3

3-(4-Hydroxyphenyl)-3-pyrrolin (3a)

3,0 g (0,01 Mol) 3-Hydroxy-(4-hydroxyphenyl)-pyrrolidin·Benzoat werden in 100 ml Wasser gelöst. Die Lösung wird dann mit konzentrierter Salzsäure auf pH 1 gebracht. Die ausgefallene Benzoesäure wird mit Ether extrahiert und die klare wäßrige Lösung eingedampft. Der feste Rückstand wird aus Ethanol umkristallisiert. Man erhält *3-(4-Hydroxyphenyl)-3-pyrrolin·HCl* in Form hellbeiger Kristalle vom Schmp. 222°C (aus Ethanol). In analoger Weise erhält man:

3-Phenyl-3-pyrrolin·HCl (3b) vom Schmp. 186—187°C (aus Propanol-2);
3-(4-Hydroxyphenyl)-1-methyl-3-pyrrolin·HCl (3c) vom Schmp. 205—207°C (aus Ethanol);
3-(3,4-Dihydroxyphenyl)-3-pyrrolin·HCl (3d) vom Schmp. 225—227°C (aus Methanol);
3-(4-Hydroxymethyl-phenyl)-3-pyrrolin·HCl (3e) vom Schmp. 206°C (aus Ethanol);
3-(3-Hydroxy-4-methyl-phenyl)-3-pyrrolin·HCl (3f) vom Schmp. 209—210°C (aus Isopropanol);
1-Methyl-3-phenyl-3-pyrrolin·HCl (3g) vom Schmp. 153—155°C (aus Isopropanol);
3-(3-Hydroxyphenyl)-3-pyrrolin·HCl (3h) vom Schmp. 187—189°C (aus Isopropanol);
3-(3-Hydroxyphenyl)-1-methyl-3-pyrrolin·HCl (3i) vom Schmp. 190—192°C (aus Ethanol);
1-Benzyl-3-phenyl-3-pyrrolin·HCl (3k) vom Schmp. 209—211°C (aus Isopropanol/Ether);
1-Benzyl-3-(4-hydroxyphenyl)-3-pyrrolin·HCl (3l) vom Schmp. 223—224°C (aus Methanol);
1-Benzyl-3-(3-hydroxyphenyl)-3-pyrrolin·HCl (3m) vom Schmp. 189—191°C (aus Ethanol)
(±)-3-(3-Hydroxyphenyl)-2-methyl-3-pyrrolin·HCl (3n) vom Schmp. 202°C (aus Propanol-2).

Die erfindungsgemäßen Verbindungen der allgemeinen Formel I bzw. II zeigen überraschende und wertvolle kardiovaskuläre Bigenschaften; insbesondere können sie zur Therapie der Herzmuskelschwäche

und des hypotonen Formenkreises eingesetzt werden.

Herzmuskelschwäche ist eine komplexe Erkrankung; sie ist multifaktoriell, progressiv und beeinträchtigt das gesamte kardiovaskuläre System. Die Behandlung dieser Erkrankung konzentriert sich auf die Stimulation der myokardialen Kontraktilität, oft in Kombination mit einer diuretischen Therapie, um das Volumenangebot des Herzens zu reduzieren.

Standard-Arzneimittel dieser Indikation sind die Herzglykoside. Ihre Anwendung wird jedoch stark eingeengt durch deren Toxizität, deren Nebenwirkungen (Arrhythmie-Erzeugung, periphere Vasokonstriktion) und deren fragwürdige Wirksamkeit bei der chronischen Behandlung.

So terten wegen der schlechten allgemeinen Verträglichkeit der Herzglykoside etwa 90 000 Vergiftungsfälle alljährlich ein, von denen der größte Teil klinisch behandelt werden muß.

Die Therapie mittels sympathomimetischer Pharmaka wird durch deren chronotrope Wirkung, deren arrhythmogene Eigenschaften und deren ungenügende orale Potenz stark begrenzt. Daher ist die Verbesserung der inotropen Therapie ein wichtiges medizinisches und soziales Anliegen.

Die erfindungsgemäßen Verbindungen der Formel I bzw. II und ihre Salze zeigen potente, positivinotrope Effekte im Tierversuch und sind in der Lage, die experimentell erzeugte Herzinsuffizienz vollständig zu kompensieren. Aufgrund der guten Verträglichkeit und das Fehlen störender Nebenwirkungen eignen sie sich vorzüglich zur Therapie der Herzmuskelschwäche und des hypotonen Syndroms. Die Einzeldosis dürfte dabei — je nach diagnostischer Erhebung — zwischen 1 und 50 mg liegen, der bevorzugte Dosierungsbereich ist 5 bis 25 mg.

Die folgenden Vergleichsversuche demonstrieren die Wirksamkeit der Verbindungen I bzw. II:

Pharmakologische Vergleichsversuche

Zur Prüfung der kardiotonen Wirkung von Substanzen ist nach Fleckenstein et al. [(Ärztl. Forschung *21*, 1—14 (1967)] die Barbituratinsuffizienz an Katzenherzen ein gut geeignetes Modell. Sie entspricht einer sogenannten Utilisationsinsuffizienz und ist damit der Insuffizienz von chronisch-hypertrophen Herzen am Menschen direkt vergleichbar.

Diederen und Kadatz [Ärztl. Forschung *24*, 149—55 (1970)] verwendeten dieses Versuchsmodell auch zur quantitativen Bestimmung der therapeutischen Breite von Herzglykosiden.

Methode

Versuchstiere waren Katzen beider Geschlechter im Gewicht von 2,7—3,9 kg. Die Tiere wurden 18 Stunden von Versuchsbeginn nüchtern gesetzt, Wasser stand ad libitum zur Verfügung. Die Anästhesie erfolgte durch intravenöse Gabe von Pentobarbital (30 mg/kg initial), anschließend wurden die Tiere zur maschinellen Beatmung tracheotomiert und es wurden die linke arteria carotis sowie die rechte arteria femoralis freigelegt.

Nach Anschließen der Tiere an eine Registriereinheit wurden gemessen:

1. EKG-Extremitätenableitung II
2. Herzfrequenz (R-Zacken-getriggert vom EKG) mit einem Pulsfrequenzmesser (in beats/min)
3. Arterieller Blutdruck in der rechten Femoralarterie mit einem Tipkatheter (in mm Hg)
4. Der linksventrikuläre Druck des Herzens mit einem Tipkatheter via linke arteria carotis (in mm Hg)
5. Die Kontraktilität des Herzens mit einem HSE-Differenzierer aus dem isometrischen Anteil der linksventrikulären Druckkurve, differenziert als $dp/dt_{max}$ (in mm Hg/sec).

Alle hämodynamischen Werte wurden fortlaufend simultan mit einem Direktschreiber aufgezeichnet.

Nach angemessener Baseline-Schreibung wurde durch Pentobarbital per infusionem eine ausgeprägte Herzinsuffizienz erzeugt. Beginnend mit 250—500 µg/0,1 ml/min wurde jedem Tier soviel Pentobarbital kontinuierlich infundiert, bis die Kontraktilität des Herzens, gemessen als $dp/dt_{max}$, um 50% gegenüber dem Ausgangswert verringert war.

Wenn dieser Punkt erreicht war, wurde nur noch soviel Pentobarbital pro Zeiteinheit infundiert, wie zur Aufrechterhaltung eines steady states der um 50% reduzierten Kontraktilität notwendig war.

In Vorversuchen wurde festgestellt, daß dieser steady state bei exakter Titration über Stunden aufrechterhalten werden konnte.

Die erfindungsgemäßen Substanzen wurden in geeigneten Dosen und Zeitabständen kumulativ intravenös appliziert, wobei ein Gesamtvolumen von 0,5 ml/kg und Dosis nicht überschritten wurde.

Die Auswertung wurde wie folgt vorgenommen:

1. Feststellung der Dosis, bei welcher die Herzinsuffizienz zu 75% behoben wurde, d.h. $dp/dt_{max}$ einen Anstieg von 75% über den Insuffizienzwert erreicht hatte. Diese Dosis wurde als Rekompensationsdosis 75 bezeichnet ($RKD_{75}$). Der 75%-Wert wurde deshalb gewählt, weil es mit dem derzeit modernsten Standard nicht möglich war, eine volle, d.h. 100%ige Rekompensation der Herzinsuffizienz herbeizuführen; mehr als 75% Rekompensationseffekt konnten mit der Vergleichssubstanz in unseren Versuchen nicht erreicht werden.

2. Berechnung eines Quotienten, der aus der intravenösen $LD_{50}$ der jeweiligen Substanz durch Division durch die $RKD_{75}$ gebildet wurde: $LD_{50}/RKD_{75}$. Diese Größe gibt annähernd die therapeutische Breite der jeweiligen Substanz wieder.

3. Berechnung der Herzfrequenz (HR) in % vom Insuffizienz-Baseline-Wert für jede applizierte Dosis.

Als Standard zum Vergleich der kardiotonen Wirkung diente Amrinon [Circ. Res. *45*, 666—77 (1979)].

# T A B E L L E

## Kennwerte von 3-Aryl-3-pyrrolin-Derivaten und der Vergleichssubstanz Amrinon

| Substanz Bsp. Nr. | $LD_{5o}$ (Maus) mg/kg i.v. | $RKD_{75}$ mg/kg i.v. | $RKD_{1oo}$ mg/kg i.v. | HR nach $RKD_{75}$ % vom AW | $\frac{LD_{5o}}{RKD_{75}}$ |
|---|---|---|---|---|---|
| 1 a | 37,5 | o,o5o | o,o7 | - 3 | 75o,o |
| 2 a | 75 | o,85o | 1,1o | + 14 | 88,2 |
| 2 c | 5o | o,28o | o,58 | + 6 | 178,6 |
| 2 d | 15o | o,135 | o,17 | + 5 | 1.111,1 |
| Amrinon | 15o | 4,ooo | wird nicht erreicht | + 11 | 37,5 |

Ergebnisse

Alle untersuchten Substanzen, einschließlich des Standards waren in der Lage, die Insuffizienz der Katzenherzen zu 75% zu rekompensieren (Tabelle).

Die Vergleichssubstanz Amrinon erreichte einen $RKD_{75}$ von 4,0 mg/kg Eine bessere Wirkung als 75% Rekompensation der Herzinsuffizienz konnte mit Amrinon nicht erreicht werden, d.h. eine restititio ad integrum war nicht möglich.

Die erfindungsgemäßen Substanzen waren hingegen in der Lage, die kardialen Insuffizienzen vollkommen zu beseitigen, d.h. 100% zu rekompensieren.

Die Herzfrequenz wird dabei von den erfindungsgemäßen Substanzen kaum beeinflußt.

In der Tabelle ist neben den Kenndaten für die untersuchten Substanzen noch die akute $LD_{50}$ angeführt, was — wie vorne erläutert — zusammen mit der $RKD_{75}$ erlaubt, einen Näherungswert für die therapeutische Breite der Substanzen anzugeben.

Die Überlegenheit der erfindungsgemäßen Substanzen genenüber dem Stand der Technik ist hiermit offensichtlich.

**Patentansprüche für die Vertragsstaaten: BE CH DE FR GB IT LI LU NL SE**

1. 3-Aryl-3-pyrrolin-Derivate der allgemeinen Formel I

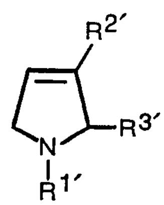

(I)

in welcher $R^{1'}$ ein Wasserstoffatom, eine geradkettige oder verzweigte Alkylgruppe mit bis zu 4 Kohlenstoffatomen oder einen Benzylrest, $R^{2'}$ einen unsubstituierten oder durch bis zu 3 gleiche oder verschiedene Substituenten, nämlich Alkyl- oder Alkoxyreste mit bis zu 4 Kohlenstoffatomen oder mit Halogenatomen, Nitro-, Trifluormethyl- oder Hydroxylgruppen substituierten Phenylrest und $R^{3'}$ ein Wasserstoffatom oder eine geradkettige oder verzweigte Alkylgruppe mit bis zu 4 Kohlenstoffatomen bedeutet, sowie deren pharmakologisch verträgliche Salz mit organischen oder anorganischen Säuren; mit Ausnahme derjenigen Verbindungen, bei denen $R^{1'}$ und $R^{3'}$ ein Wasserstoffatom und gleichzeitig $R^{2'}$ einen unsubstituierten Phenylrest oder einen 3-Trifluormethylphenylrest darstellt.

2. 1-Methyl-3-phenyl-3-pyrrolin und dessen pharmakologisch verträgliche Salze mit organischen oder anorganischen Säuren.

3. Verfahren zur Herstellung von Verbindungen gemäß Anspruch 1 dadurch gekennzeichnet, daß man in an sich bekannter Weise entweder

a) eine Verbindung der allgemeinen Formel III

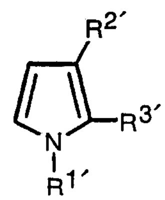

(III),

in welcher die Reste $R^{1'}$, $R^{2'}$ und $R^{3'}$ die obengenannte Bedeutung haben, mittels Zink und einer Mineralsäure reduziert oder

b) eine Verbindung der allgemeinen Formel IV

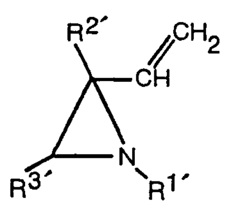

(IV),

in welcher $R^{1'}$, $R^{2'}$ und $R^{3'}$ die obengenannte Bedeutung haben, mit Bromwasserstoffsäure umsetzt, oder

c) eine Verbindung der allgemeinen Formel V

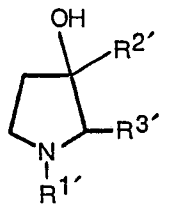

(V),

in welcher $R^{1'}$, $R^{2'}$ und $R^{3'}$ die obengenannte Bedeutung haben, mit einer Mineralsäure dehydratisiert und die erhaltenen Verbindungen der allgemeinen Formel I gewünschtenfalls in an sich bekannter Weise in deren pharmakologisch verträgliche Salze überführt.

4. Arzneimittel mit kardiovaskulärer Wirkung, dadurch gekennzeichnet, daß es neben üblichen Hilfs- und Trägerstoffen mindestens eine Verbindung der allgemeinen Formel II

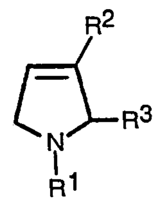

(II),

in welcher $R^1$ ein Wasserstoffatom, eine geradkettige oder verzweigte Alkylgruppe mit bis zu 4 Kohlenstoffatomen oder einen Benzylrest, $R^2$ einen unsubstituierten oder durch bis zu 3 gleiche oder verschiedene Substituenten, nämlich Alkyl- oder Alkoxyreste mit bis zu 4 Kohlenstoffatomen oder mit Halogenatomen, Nitro-, Trifluormethyl- oder Hydroxylgruppen substituierten Phenylrest und $R^3$ ein Wasserstoffatom oder eine geradkettige oder verzweigte Alkylgruppe mit bis zu 4 Kohlenstoffatomen bedeutet,

oder deren pharmakologisch verträgliche Salze mit organischen oder anorganischen Säuren enthält.

5. Arzneimittel gemäß Anspruch 4, dadurch gekennzeichnet, daß es als Wirkstoff 1-Methyl-3-phenyl-3-pyrrolin und/oder 3-Phenyl-3-pyrrolin oder deren pharmakologisch verträgliche Salze enthält.

**Patentansprüche für die Vertragsstaat: AT**

1. Verfahren zur Herstellung von 3-Aryl-3-pyrrolin-Derivate der allgemeinen Formel I

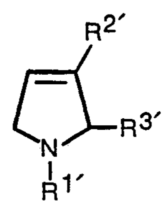

(I)

in welcher $R^{1'}$ ein Wasserstoffatom, eine geradkettige oder verzweigte Alkylgruppe mit bis zu 4 Kohlenstoffatomen oder einen Benzylrest, $R^{2'}$ einen unsubstituierten oder durch bis zu 3 gleiche oder verschiedene Substituenten, nämlich Alkyl- oder Alkoxyreste mit bis zu 4 Kohlenstoffatomen oder mit Halogenatomen, Nitro-, Trifluormethyl- oder Hydroxylgruppe substituierten Phenylrest und $R^{3'}$ ein Wasserstoffatom oder eine geradkettige oder verzweigte Alkylgruppe mit bis zu 4 Kohlenstoffatomen bedeutet, sowie deren pharmakologisch verträgliche Salze mit organischen oder anorganischen Säuren; mit Ausnahme derjenigen Verbindungen, bei denen $R^{1'}$ und $R^{3'}$ ein Wasserstoffatom und gleichzeitig $R^{2'}$ einen unsubstituierten Phenylrest oder einen 3-Trifluormethylphenylrest darstellt, dadurch gekennzeichnet, daß man in an sich bekannter Weise entweder

a) eine Verbindung der allgemeinen Formel III

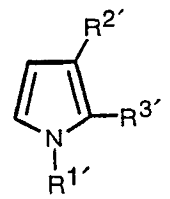

(III),

in welcher die Reste $R^{1'}$, $R^{2'}$ und $R^{3'}$ die obengenannte Bedeutung haben, mittels Zink und einer Mineralsäure reduziert oder

b) eine Verbindung der allgemeinen Formel IV

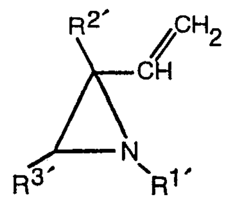

(IV),

in welcher $R^{1'}$, $R^{2'}$ und $R^{3'}$ die obengenannte Bedeutung haben, mit Bromwasserstoffsäure umsetzt, oder

c) eine Verbindung der allgemeinen Formel V

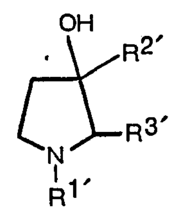

(V),

in welcher $R^{1'}$, $R^{2'}$ und $R^{3'}$ die obengenannte Bedeutung haben, mit einer Mineralsäure dehydratisiert und die erhaltenen Verbindungen der allgemeinen Formel I gewünschtenfalls in an sich bekannter Weise in deren pharmakologisch verträgliche Salze überführt.

2. Verfahren gemäß Anspruch 1 zur Herstellung von 1-Methyl-3-phenyl-3-pyrrolin und dessen pharmakologisch verträgliche Salze mit organischen oder anorganischen Säuren.

**Revendications pour les Etats contractants: BE CH DE FR GB IT LI LU NL SE**

1. Dérivés de 3-aryl-3-pyrroline de formule générale I:

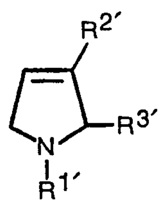

(I)

dans laquelle:

$R^{1'}$ représente un atome d'hydrogène, un groupe alkyle à chaîne linéaire ou ramifiée renfermant jusqu'à 4 atomes de carbone ou un reste benzyle;

$R^{2'}$ représente un reste phényle non substitué ou substitué par jusqu'à trois substituants identiques ou différents, à savoir des restes alkyle ou alkoxy renfermant jusqu'à 4 atomes de carbone ou par des atomes d'halogène, des groupes nitro, trifluorométhyle ou hydroxyle; et

$R^{3'}$ représente un atome d'hydrogène ou un groupe alkyle à chaîne linéaire ou ramifiée renfermant jusqu'à 4 atomes de carbone,

ainsi que leurs sels pharmacologiquement compatibles avec des acides organiques ou minéraux; à l'exception des dérivés dans lesquels $R^{1'}$ et $R^{3'}$ représentent un atome d'hydrogène et $R^{2'}$ représente en même temps un reste phényle non substitué ou un reste 3-trifluorométhylphényle.

2. 1-méthyl-3-phényl-3-pyrroline et leurs sels pharmacologiquement compatibles avec des acides organiques ou minéraux.

3. Procédé de préparation de dérivés selon la revendication 1, caractérisé en ce que, d'un façon connue en soi, on réalise l'une ou l'autre des étapes suivantes:

(a) on réduit un dérivé de formule générale III:

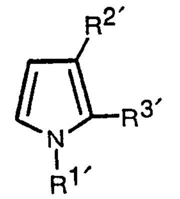

(III),

dans laquelle les restes $R^{1'}$, $R^{2'}$ et $R^{3'}$ présentent la signification indiquée ci-dessus, au moyen de zinc et d'un acide minéral; ou

(b) ou fait réagir un dérivé de formule générale IV:

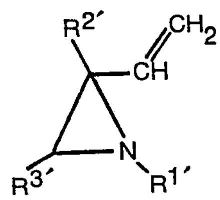

(IV),

dans laquelle $R^{1'}$, $R^{2'}$ et $R^{3'}$ présentent la signification indiquée ci-dessus, avec de l'acide bromhydrique; ou

(c) on déshydrate un dérivé de formule générale V:

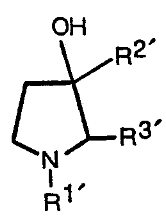

(V),

dans laquelle $R^{1'}$, $R^{2'}$ et $R^{3'}$ présentent la signification indiquée ci-dessus,

à l'aide d'un acide minéral,

et l'on fait passer les dérivés de formule générale I obtenus, le cas échéant d'une façon connue en soi, en leurs sels pharmacologiquement compatibles.

4. Médicaments à effet cardiovasculaire caractérisés en ce que, outre les adjuvants et supports usuels, ils contiennent au moins un dérivé de formule générale II:

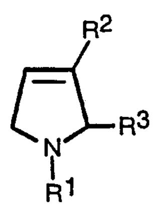

(II),

dans laquelle:

$R^1$ représente un atome d'hydrogène, un groupe alkyle à chaîne linéaire ou ramifiée renfermant jusqu'à 4 atomes de carbone ou un reste benzyle;

$R^2$ représente un radical phényle non substitué ou substitué par jusqu'à trois substituants identiques

ou différents, à savoir des restes alkyle ou alkoxy renfermant jusqu'à 4 atomes de carbone, ou par des atomes d'halogène, des groupes nitro, trifluorométhyle ou hydroxyle; et

$R^3$ représente un atome d'hydrogène ou un groupe alkyle à chaîne linéaire ou ramifiée renfermant jusqu'à 4 atomes de carbone,

ou leurs sels pharmacologiquement compatibles avec des acides organiques ou minéraux.

5. Médicament selon la revendication 4, caractérisé en ce qu'il contient comme substance active la 1-méthyl-3-phényl-3-pyrroline et/ou la 3-phényl-3-pyrroline ou leurs sels pharmacologiquement compatibles.

**Revendications pour l'Etat contractant: AT**

1. Procédé de préparation de dérivés de 3-aryl-3-pyrroline de formule générale I:

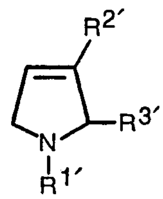

(I)

dans laquelle:

$R^{1'}$ représente un atome d'hydrogène, un groupe alkyle à chaîne linéaire ou ramifiée renfermant jusqu'à 4 atomes de carbone ou un reste benzyle;

$R^{2'}$ représente un reste phényle non substitué ou substitué par jusqu'à trois substituants identiques ou différents, à savoir des restes alkyle ou alkoxy renfermant jusqu'à 4 atomes de carbone ou par des atomes d'halogène, des groupes nitro, trifluorométhyle ou hydroxyle; et

$R^{3'}$ représente un atome d'hydrogène ou un groupe alkyle à chaîne linéaire ou ramifiée renfermant jusqu'à 4 atomes de carbone,

ainsi que leurs sels pharmacologiquement compatibles avec des acides organiques ou minéraux; à l'exception des dérivés dans lesquels $R^{1'}$ et $R^{3'}$ représentent un atome d'hydrogène et $R^{2'}$ représente en même temps un reste phényle non substitué ou un reste 3-trifluorométhylphényle;

caractérisé en ce que, d'une façon connue en soi, on réalise l'une ou l'autre des étapes suivantes:

(a) on réduit un dérivé de formule générale III:

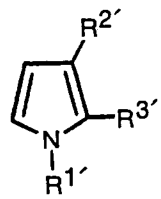

(III),

dans laquelle les restes $R^{1'}$, $R^{2'}$ et $R^{3'}$ présentent la signification indiquée ci-dessus, au moyen de zinc et d'un acide minéral; ou

(b) ou fait réagir un dérivé de formule générale IV:

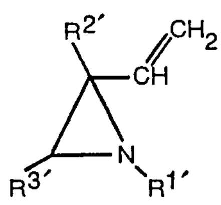

(IV),

dans laquelle $R^{1'}$, $R^{2'}$ et $R^{3'}$ présentent la signification indiquée ci-dessus, avec de l'acide bromhydrique; ou

(c) on déshydrate un dérivé de formule générale V:

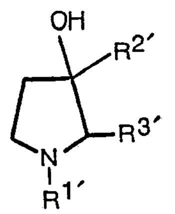

(V),

dans laquelle $R^{1'}$, $R^{2'}$ et $R^{3'}$ présentent la signification indiquée ci-dessus, à l'aide d'un acide minéral,

et l'on fait passer les dérivés de formule générale I obtenus, le cas échéant d'une façon connue en soi, en leurs sels pharmacologiquement compatibles.

2. Procédé selon la revendication 1 de préparation de 1-méthyl-3-phényl-3-pyrroline et de leurs sels pharmacologiquement compatibles avec des acides organiques ou minéraux.

**Claims for the Contracting States: BE CH DE FR GB IT LI LU NL SE**

1. 3-aryl-3-pyrroline derivatives of the general formula I,

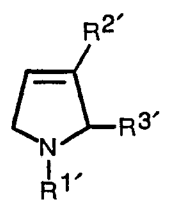

(I)

in which $R^{1'}$ denotes a hydrogen atom, a straight-chain or branched alkyl group with up to 4 carbon atoms or a benzyl radical, $R^{2'}$ denotes an unsubstituted phenyl radical or a phenyl radical substituted by up to 3 identical or different substituents, namely alkyl or alkoxy radicals with up to 4 carbon atoms or with halogen atoms, nitro, trifluoromethyl or hydroxyl groups, and $R^{3'}$ denotes a hydrogen atom or a straight-chain or branched alkyl group with up to 4 carbon atoms, and pharmacologically acceptable salts thereof with organic or inorganic acids; with the exception of those compounds in which $R^{1'}$ and $R^{3'}$ represent a hydrogen atom, and $R^{2'}$ represents at the same time an unsubstituted phenyl radical or a 3-trifluoromethyl phenyl radical.

2. 1-methyl-3-phenyl-3-pyrroline and pharmacologically acceptable salts thereof with organic or inorganic acids.

3. Process for the preparation of compounds according to claim 1, characterised in that, in a manner known per se, either

a) a compound of the general formula III,

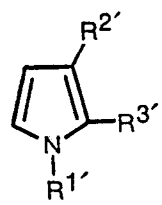

(III),

in which the radicals $R^{1'}$, $R^{2'}$ and $R^{3'}$ have the above meaning,
is reduced by means of zinc and a mineral acid, or

b) a compound of the general formula IV,

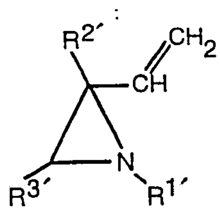

(IV),

in which $R^{1'}$, $R^{2'}$ and $R^{3'}$ have the above meaning, is caused to react with hydrobromic acid, or

c) a compound of the general formula V,

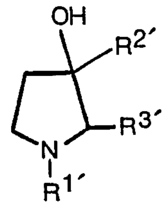

(V),

in which $R^{1'}$, $R^{2'}$ and $R^{3'}$ have the above meaning, is dehydrated with a mineral acid, and the obtained compounds of the general formula I are converted, as desired, in a manner known per se, into pharmacologically acceptable salts thereof.

4. Medicament with a cardiovascular effect, characterised in that it contains, besides the usual adjuvant and carrier substances, at least one compound of the general formula II,

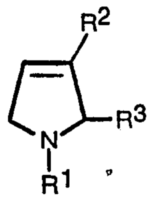

(II),

in which $R^1$ denotes a hydrogen atom, a straight-chain or branched alkyl group with up to 4 carbon atoms or a benzyl radical, $R^2$ denotes an unsubstituted phenyl radical or a phenyl radical substituted by up to 3 identical or different substituents, namely alkyl or alkoxy radicals with up to 4 carbon atoms or with halogen atoms, nitro, trifluoromethyl or hydroxyl groups, and $R^3$ denotes a hydrogen atom or a straight-chain or branched alkyl group with up to 4 carbon atoms, or contains pharmacologically acceptable salts thereof with organic or inorganic acids.

5. Medicament according to claim 4, characterised in that it contains as the effective substance 1-methyl-3-phenyl-3-pyrroline and/or 3-phenyl-3-pyrroline or pharmacologically acceptable salts thereof.

**Claims for the Contracting State: AT**

1. Process for the preparation of 3-aryl-3-pyrroline derivatives of the general formula I,

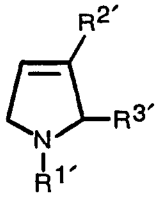

(I)

in which $R^{1'}$ denotes a hydrogen atom, a straight-chain or branched alkyl group with up to 4 carbon atoms or a benzyl radical, $R^{2'}$ denotes an unsubstituted phenyl radical or a phenyl radical substituted by up to 3 identical or different substituents, namely alkyl or alkoxy radicals with up to 4 carbon atoms

or with halogen atoms, nitro, trifluoromethyl or hydroxyl groups, and $R^{3'}$ denotes a hydrogen atom or a straight-chain or branched alkyl group with up to 4 carbon atoms, and pharmacologically acceptable salts thereof with organic or inorganic acids; with the exception of those compounds in which $R^{1'}$ and $R^{3'}$ represent a hydrogen atom, and $R^{2'}$ represents at the same time an unsubstituted phenyl radical or a 3-trifluoromethyl phenyl radical,

characterised in that, in a manner known per se, either

a) a compound of the general formula III,

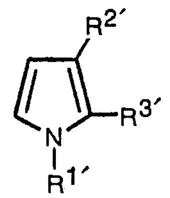

(III),

in which the radicals $R^{1'}$, $R^{2'}$ and $R^{3'}$ have the above meaning, is reduced by means of zinc and a mineral acid, or

b) a compound of the general formula IV,

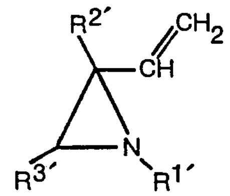

(IV),

in which $R^{1'}$, $R^{2'}$ and $R^{3'}$ have the above meaning, is caused to react with hydrobromic acid, or

c) a compound of the general formula V,

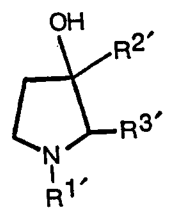

(V),

in which $R^{1'}$, $R^{2'}$ and $R^{3'}$ have the above meaning, is dehydrated with a mineral acid, and the obtained compounds of the general formula I are converted, as desired, in a manner known per se, into pharmacologically acceptable salts thereof.

2. Process according to claim 1 for the preparation of 1-methyl-3-phenyl-3-pyrroline and pharmacologically acceptable salts thereof with organic or inorganic acids.